Europäisches Patentamt

European Patent Office

Office européen des brevets

(11) Publication number: **0 156 352**
**B1**

(12) **EUROPEAN PATENT SPECIFICATION**

(45) Date of publication of patent specification: 03.08.88

(51) Int. Cl.⁴: **C 07 C 149/36,** C 07 C 148/00

(21) Application number: 85103534.5

(22) Date of filing: 25.03.85

(54) Preparation of (alkylthio-,cycloalkythio- or arylthio)-phenols.

(30) Priority: 26.03.84 US 593562

(43) Date of publication of application:
02.10.85 Bulletin 85/40

(45) Publication of the grant of the patent:
03.08.88 Bulletin 88/31

(84) Designated Contracting States:
AT BE CH DE FR GB IT LI LU NL SE

(56) References cited:
US-A-4 324 920

(73) Proprietor: **ETHYL CORPORATION**
**Ethyl Tower 451 Florida Boulevard**
**Baton Rouge Louisiana 70801 (US)**

(72) Inventor: **Ranken, Paul Frederick**
**10345 Westwood Avenue Baton Rouge**
**Louisiana 70809 (US)**

(74) Representative: **Dipl.-Ing. Schwabe, Dr. Dr.**
**Sandmair, Dr. Marx**
**Stuntzstrasse 16**
**D-8000 München 80 (DE)**

Courier Press, Leamington Spa, England.

## Description

This invention relates to a process for preparing (alkylthio-, cycloalkylthio- or arylthio)-phenols by the redistribution· of other (alkylthio-, cycloalkylthio- or arylthio)-phenols.

As disclosed in U.S. Patents 2,923,743 (Delfs et al.) and 4,324,920 (McKinnie et al.), it is known that 2-(hydrocarbylthio)phenols are useful as pharmaceutical and agricultural intermediates in the preparation of hypotensive drugs and agricultural chemicals, such as plant protection agents, herbicides, pesticides· and the like, and that they can be prepared by reacting appropriate phenols with hydrocaryl disulfides in the presence of aluminum compounds, such as the aluminum phenoxide catalyst of McKinnie et al.

Unfortunately, these 2-(hydrocarbylthio)phenol syntheses also lead to the formation of significant quantities of 4-(hydrocarbylthio)phenols as well as small amounts of poly(hydrocarbylthio)phenols. It would therefore be desirable to be able to convert at least a portion of these by-products to the more desirable 2-(hydrocarbylthio)phenols.

In other situations, it is desired to prepare another particular (hydrocarbylthio)phenol, e.g., a 4-(hydrocarbylthio)phenol; also in this case by-product (hydrocarbylthio)phenols are formed and it would be desirable to convert at least a portion of those (hydrocarbylthio)phenol by-products to the desired (hydrocarbylthio)phenol.

It has now been found that according to this invention the desired object can be achieved by a process for preparing (alkylthio-, cycloalkylthio- or arylthio)phenols by the redistribution of other (alkylthio, cycloalkylthio or arylthio-)phenols which comprises heating one or more of said phenols selected from 2-, 4- and poly(alkylthio, cycloalkylthio or arylthio-)phenols in the presence of a catalytic amount of an aluminum phenoxide or a precursor thereof to redistribute the (alkylthio, cycloalkylthio or arylthio-) phenol starting material.

This inventive process can be conducted very easily and economically and provides the desired (alkylthio, cycloalkylthio or arylthio-)phenol in enhanced yields. Furthermore, it is useful to convert useless by-products into commercially valuable products.

(Alkylthio, cycloalkylthio or arylthio-)phenols useful as starting materials in the practice of the invention are compounds having one or more of said groups attached to an aromatic ring which optionally bears one or more inert substituents in the positions unsubstituted by said groups. These (alkylthio, cyclalkylthio or arylthio-)phenols are generally 4-(alkylthio, cycloalkylthio or arylthio-)phenols, 2-(alkylthio, cycloalkylthio or arylthio-)phenols, 2,4-di(alkylthio, cycloalkylthio- or arylthio-)phenols, 2,6-di(alkylthio, cycloalkylthio or arylthio-) phenols and/or 2,4,6-tri(alkylthio, cycloalkylthio or arylthio-)phenols.

As in McKinnie et al. the alkylthio, cycloalkylthio- or arylthio groups of the (alkylthio, cycloalkylthio or arylthio)phenols may be methylthio, ethylthio, butylthio, cyclopentylthio, cyclohexylthio, benzylthio-, p-tolylthio, p-chlorophenylthio, etc.; but they are preferably alkylthio groups, most preferably alkylthio groups containing 1,6-carbons.

The phenolic substrate of the (alkylthio-, cycloalkylthio- or arylthio)-phenol may be a mono- or polynuclear hydroxy aromatic compound, optionally bearing one or more inert substituents, such as the phenols taught in McKinnie et al. and other such phenols. In general, these phenolic substrates are compounds containing one or more simple or fused aromatic rings, such as benzene, naphthalene, indene, benzofuran, biphenyl, etc., rings, bearing one or more hydroxy substituents as well as any optionally substituents on the same different rings, including such compounds wherein two aromatic rings are linked by a divalent group, such as an alkylidene, alkylene, oxygen, or sulfur linkage, e.g., bisphenol A, 4,4'-methylenediphenol, etc. However, the phenolic substrate is preferably a mononuclear, monohydroxy phenol, such as phenol itself, optionally bearing one or more alkyl side chains and up to two aryl-chloro substituents. Thus, the preferred (alkylthio-, cycloalkylthio- or arylthio)phenol starting materials are optionally-substituted mononuclear, monohydroxy 2-,, 4-, 2,4-, 2,6-, and/or 2,4,6- mono-, di-, and/or tri(alkylthio)phenols having 1—6 carbons in the alkyl groups.

The aluminum phenoxide catalyst used in the practice of this invention can be formed in various ways. For example it can be formed by contacting aluminum metal, preferably in the form of turnings, powders, particles and the like with the phenol at elevated temperatures (e.g. 100—200°C) until cessation of hydrogen evolution. Another way of forming the catalyst used in this invention is by simply contacting an aluminum alkyl such as triethylaluminum with the phenol. Still another method of forming the catalyst used herein involves contacting aluminum chloride (i.e. AlCl$_3$) and the phenol at elevated temperatures, e.g. between 100° and 200°C and purging the system of the hydrogen chloride generated during the preparation of the phenoxide catalyst. The source of the aluminum phenoxide catalyst does not materialy affect the reaction and other methods known in the art for the formation of these compounds can be successfully employed herein. Exemplary methods for preparing aluminum phenoxide catalysts useful in the process of this invention appear in U.S. Pat. Nos. 2,831,898; 2,923,745 and 3,200,157. The preferred catalyst is aluminum tri-phenoxide, although the di-phenoxide catalyst, e.g., di-phenoxy aluminum chloride which may be formed in the reaction of aluminum chloride and phenol, can also be used in the practice of this invention.

As in McKinnie et al., the aluminum phenoxide can be prepared by contacting aluminum, an aluminum alkyl (e.g., triethylaluminum), or aluminum chloride with a phenol, desirably the (alkylthio-, cycloalkylthio- or arylthio)phenol starting material, at an elevated temperature, e.g. 100—200°C. In a preferred

2

embodiment of the invention, the catalyst is formed in situ to form the phenoxide corresponding to the (alkylthio-, cycloalkylthio- or arylthio)phenol starting material.

The process of the invention is conducted by contacting the (alkylthio-, cycloalkylthio- or arylthio)phenol with a catalytic amount, e.g., 0,01—0,5, preferably 0,01—0,2 mol per mol of (alkylthio-, cycloalkylthio- or arylthio)phenol, of the aluminum phenoxide of a precursor thereof and heating the reaction mixture at a suitable temperature, e.g., 100—300°C, preferably 120—200°C, until the (alkylthio-, cycloalkylthio- or aryl-thio) groups have been redistributed and the desired (alkylthio-, cycloalkylthio- or arylthio)phenol has been formed. If desired, the reaction can be conducted in the presence of an added phenol, such as phenol, p-cresol, etc., which can be useful in reacting with (alkylthio-, cycloalkylthio- or arylthio)phenol when a (alkylthio-, cycloalkylthio- or arylthio)phenol containing fewer alkylthio-, cycloalkylthio- or arylthio groups is desired.

While the process of this invention can be carried out without the use of solvents or diluents they may nevertheless be used, if desired, so long as they are inert to the reaction disclosed herein. The process is preferably carried out under substantially anhydrous conditions, but conditions wherein trace or small amounts of water are present can be used. Accordingly, an inert atmosphere is normally employed in the present process.

After completion of the redistribution reaction, the desired (alkylthio-, cycloalkylthio- or arylthio)phenol can be isolated by fractionation. Alternatively, the reaction mixture can be reacted with a suitable disulfide to provide additional quantities of the desired (alkylthio-, cycloalkylthio or arylthio)phenol.

This invention is illustrated by the following examples of preferred embodiments of the invention without, however, being restricted thereto.

Example I

A suitable reaction vessel was charged, under nitrogen, with 3,1 g (0,022 mole) of pure 4-(methylthio)phenol that had been recrystallized from toluene and then with 0,20 ml (0,0015 mol) of triethylaluminum. The reaction was stirred at 170—180°C under nitrogen for about 18 hours. A sample of the reaction mixture was diluted with ether and extracted with 6N HCl and then saturated aqueous NaCl. Gas chromatographic analysis showed:

| Ingredient | Area % |
| --- | --- |
| Phenol | 23 |
| 2-(methylthio)phenol | 30 |
| 4-(methylthio)phenol | 11 |
| 3-(methylthio)phenol | 10 |
| 2,4-di(methylthio)phenol | 12 |
| 2,6-di(methylthio)phenol | 10 |
| 2,4,6-tri(methylthio)phenol | 2 |
| Others | 2 |

Example II

In a nitrogen atmosphere, 0,3 ml (0,0022 mol) of triethylaluminum was added to 4,67 g (0,033 mol) of 2-(methylthio)phenol. The clear yellow solution was stirred at 170—180°C for 18,5 hours to give a clear cherry-red solution which was diluted with 10 ml of diethyl ether and extracted with 10 ml of 6N HCl and then with 10 ml of saturated aqueous NaCl. Gas chromatographic analysis of the sodium sulfate-dried organics indicated the following normalized composition:

| Ingredient | Area % |
| --- | --- |
| Phenol | 22 |
| 2-(methylthio)phenol | 34 |
| 4-(methylthio)phenol | 13 |
| 3-(methylthio)phenol | 3 |
| 2,4-di(methylthio)phenol | 14 |
| 2,6-di(methylthio)phenol | 9 |
| 2,4,6-tri(methylthio)phenol | 2 |
| Others | 3 |

Example III

A suitable reaction vessel was charged with 4,0 g (0,020 mol) of 4-methyl-2,6-di(methylthio)phenol and 2,16 g (0,02 mol) of p-cresol, to which 0,18 ml (0,00135 mol) of triethylaluminum was added in a nitrogen atmosphere. The yellow solution was stirred at 160—180°C for 18 hours. After cooling, the reaction mixture was diluted with 10 ml of toluene, extracted with 10 ml 6N HCl and 10 ml saturated NaCl, dried over sodium sulfate, and filtered. Removal of the solvent with a rotary evaporator gave a reddish liquid which gas chromatographic analysis showed to contain:

3

**0 156 352**

| Ingredient | Area % |
|---|---|
| p-cresol | 19 |
| 4-methyl-2-(methylthio)phenol | 47 |
| 4-methyl-3-(methylthio)phenol | 4 |
| 4-methyl-2,6-di(methylthio)phenol | 29 |
| Others | 1 |

Gas chromatographic analysis using dodecane as an internal standard showed that the product was 14% by weight of p-cresol, 40% by weight of 4-methyl-2-(methylthio)phenol, and 27% by weight of 4-methyl-2,6-di(methylthio)phenol.

**Claims**

1. A process for preparing (alkylthio-, cycloalkylthio-, or arylthio) phenols by the redistribution of other (alkylthio-, cycloalkylthio- or arylthio)phenols which comprises heating one or more of said phenols selected from 2-, 4-, and poly(alkylthio-, cycloalkylthio- or arylthio) phenols in the presence of a catalytic amount of an aluminum phenoxide or a precursor thereof to redistribute the (alkylthio-, cycloalkylthio- or arylthio)phenol starting material.

2. The process as claimed in Claim 1 wherein the starting material is a 2-(alkylthio-, cycloalkylthio- or arylthio)phenol.

3. The process as claimed in Claim 1 wherein the starting material is a 4-(alkylthio-, cycloalkylthio- or arylthio)phenol.

4. The process as claimed in Claim 1, wherein the starting material is a mixture of (alkylthio-cycloalkylthio- or arylthio)phenols.

5. The process as claimed in Claims 1 to 4 wherein the (alkylthio)phenol is a mononuclear, monohydroxy (alkylthio)phenol having 1—6 carbons in the alkyl group.

6. The process as claimed in Claim 5 wherein the (alkylthio)phenol is a (methylthio)phenol.

7. The process as claimed in Claims 1 to 6 wherein the aluminum phenoxide is generated in situ.

8. The process as claimed in Claims 1 to 7 wherein the reaction is conducted at a temperature of 100—300°C, preferably 120—200°C.

9. The process as claimed in Claims 1 to 8 wherein the aluminum phenoxide or the precursor thereof is used in an amount of 0,01 to 0,5, preferably 0,01—0,2 mol per mol (alkylthio-, cycloalkylthio- or arylthio)phenol.

10. The process as claimed in Claims 1 to 9 wherein the reaction is conducted in the presence of an added phenol preferably phenol per se or p-cresol.

**Patentansprüche**

1. Verfahren zur Herstellung von (Alkylthio-, Cycloalkylthio- oder Arylthio)-phenole durch Neuverteilung von anderen (Alkylthio-, Cycloalkylthio- oder Arylthio)-phenolen, bei dem man eines oder mehrere dieser Phenole, ausgewählt aus 2-, 4- und Poly-(alkylthio-, cycloalkylthio- oder arylthio)-phenol in Gegenwart einer katalytischen Menge eines Aluminiumphenoxids oder einer Vorstufe davon erwärmt, um das (Alkylthio- Cycloalkylthio- oder Arylthio)-phenol-Ausgangsmaterial neu zu verteilen.

2. Verfahren nach Anspruch 1, worin das Ausgangmaterial ein 2-(Alkylthio-, Cycloalkylthio- oder Arylthio)-phenol ist.

3. Verfahren nach Anspruch 1, worin das Ausgangsmaterial ein 4-(Alkylthio-, Cycloalkylthio- oder Arylthio)-phenol ist.

4. Verfahren nach Anspruch 1, worin das Ausgangsmaterial eine Mischung aus (Alkylthio-, Cycloalkylthio- oder Arylthio)-phenolen ist.

5. Verfahren nach Anspruch 1 bis 4, worin das (Alkylthio)-phenol ein einkerniges, Monohydroxy-(alkylthio)-phenol mit 1—6 Kohlenstoffatomen in der Alkylgruppe ist.

6. Verfahren nach Anspruch 5, worin das (Alkylthio)-phenol ein (Methylthio)-phenol ist.

7. Verfahren nach Anspruch 1 bis 6, worin das Aluminiumphenoxid in situ erzeugt wird.

8. Verfahren nach Anspruch 1 bis 7, worin die Reaktion bei einer Temperatur von 100 bis 300°C, vorzugsweise 120 bis 200°C, durchgeführt wird.

9. Verfahren nach Anspruch 1 bis 8, worin das Aluminiumphenoxid oder die Vorstufe davon in einer Menge von 0,01 bis 0,5, vorzugsweise 0,01 bis 0,2 Mol pro Mol (Alkylthio-, Cycloalkylthio- oder Arylthio)-phenol verwendet wird.

10. Verfahren nach Anspruch 1 bis 9, worin die Reaktion in Gegenwart von zugegebenem Phenol, vorzugsweise Phenol als solchem oder para-Kresol, durchgeführt wird.

**Revendications**

1. Procédé de préparation de (alkylthio-, cycloalkylthio- ou arylthio)phénols par la redistribution

4

d'autres (alkyltio-, cycloalkylthio- ou arylthio)phénols, qui consiste à chauffer un ou plusieurs de ces phénols choisis entre des 2-, 4-, et poly(alkylthio-, cycloalkylthio- ou arylthio)phénols en présence d'une quantité catalytique d'un phénate d'aluminium ou d'un précurseur de phénate d'aluminium pour redistribuer le (alkylthio-, cycloalkylthio-, ou arylthio)phénol de départ.

2. Procédé suivant la revendication 1, dans lequel la matière de départ est un 2-(alkylthio-, cycloalkylthio- ou arylthio)phénol.

3. Procédé suivant la revendication 1, dans lequel la matière de départ est un 4-(alkylthio-, cycloalkylthio- ou arylthio)phénol.

4. Procédé suivant la revendication 1, dans lequel la matière de départ est un mélange de (alkylthio-, cycloalkylthio- ou arylthio)phénols.

5. Procédé suivant les revendications 1 à 4, dans lequel le (alkylthio)phénol est un (alkylthio)phénol mono-hydroxylique mono-nucléaire ayant 1 à 6 atomes de carbone dans le groupe alkyle.

6. Procédé suivant la revendication 5, dans lequel le (alkylthio)phénol est un (méthylthio)phénol.

7. Procédé suivant les revendications 1 à 6, dans lequel le phénate d'aluminium est engendré in situ.

8. Procédé suivant les revendications 1 à 7, dans lequel la réaction est conduite à une température de 100 à 300°C, de préférence de 120 à 200°C.

9. Procédé suivant les revendications 1 à 8, dans lequel le phénate d'aluminium ou son précurseur est utilisé en une quantité de 0,01 à 0,5 de préférence de 0,01 à 0,2 mole par mole de (alkylthio-, cycloalkylthio- ou arylthio)phénol.

10. Procédé suivant les revendications 1 à 9, dans lequel la réaction est conduite en présence d'un phénol ajouté, de préférence le phénol proprement dit ou le p-crésol.